# EUROPEAN PATENT SPECIFICATION

(11) **EP 1 394 183 B1**
(45) Date of publication and mention of the grant of the patent: **12.08.2009**
(21) Application number: 03292060.5
(22) Date of filing: 20.08.2003
(51) Int. Cl.: C07K 16/06, C07K 16/00, A61K 39/395

(54) **Purification of polyreactive autoantibodies and uses thereof**
Reinigung von polyreaktiven Autoantikörpern und deren Verwendungen
Purification d'autoanticorps polyréactifs et leurs utilisations

(30) Priority: 20.08.2002 US 404416 P
(43) Date of publication of application: 03.03.2004
(73) Proprietor: Hema-Quebec, Sainte-Foy, Québec G1V 4M3 (CA)
(72) Inventor: Lemieux, Réal, G1X 4Y9 Ste-Foy Quebec (CA); Lamoureux, Josée, G1H 5H5 Charlesbourg Quebec (CA)
(74) Representative: Ahner, Francis

(56) References cited:
- EP-A- 1 059 088
- DE-A- 19 900 503
- US-A- 4 256 631
- VASSILEV TCHAVDAR L ET AL: "Variable Region-Connected, Dimeric Fraction of Intravenous immunoglobulin Enriched in Natural Autoantibodies" JOURNAL OF AUTOIMMUNITY, vol. 8, no. 3, 1995, pages 405-413, XP002265834 ISSN: 0896-8411
- VASSILEV T L ET AL: "Inhibition of cell adhesion by antibodies to Arg-Gly-Asp(RGD) in normal immunoglobulin for therapeutic use (intravenous immunoglobulin, IVIg)" BLOOD, W.B.SAUNDERS COMPAGNY, ORLANDO, FL, US, vol. 93, no. 11, 1999, pages 3624-3631, XP002952889 ISSN: 0006-4971
- KAVERI SRINI ET AL: "Antibodies to a conserved region of HLA class I molecules, capable of modulating CD8 T cell-mediated function, are present in pooled normal immunoglobulin for therapeutic use" JOURNAL OF CLINICAL INVESTIGATION, vol. 97, no. 3, 1996, pages 865-869, XP002265835 ISSN: 0021-9738
- LAMOUREUX ET AL INTERNATIONAL IMMUNOLOGY vol. 16, no. 7, 2004, pages 929 - 936
- LAMOUREUX ET AL BLOOD vol. 101, no. 4, 15 February 2003, pages 1660 - 1662
- LEMIEUX; BAZIN CURRENT PHARMACEUTICAL DESIGN vol. 12, 2006, pages 173 - 179

## Description

### BACKGROUND OF THE INVENTION

### (a) Field of the Invention

This invention relates to a method to purify autoantibodies from therapeutic intravenous immunoglobulin preparations (IVIg); autoantibodies; IVIg free of autoantibodies, pharmaceutical compositions, therapeutical uses and method of treatments thereof.

### (b) Description of Prior Art

Intravenous immunoglobulins (IVIg) are widely used in the supportive therapy of immunodeficient patients and in the treatment of a wide variety of chronic autoimmune and inflammatory diseases such as immune thrombocypotenia purpura (ITP) and systemic lupus erythematosus (SLE)^{1,2}. The mechanisms of action of IVIg in most autoimmune and inflammatory diseases are still unclear and are attracting much interest due to the increasing IVIg utilization and the possibility of IVIg shortages caused by the limitations in the volume of human source plasma that can be collected from donors ³. The proposed mechanisms of action of IVIg are diversified and include the inhibition of phagocytosis and the modulation of the complement system ^{1,2}. The inhibition of phagocytosis has been observed in diseases such as ITP in which platelets opsonized by the pathogenic autoantibodies are no longer phagocyted shortly after the infusion of IVIg (reviewed in ⁴) Several mechanisms of inhibition of phagocytosis by IVIg have been proposed and include direct competitive blockage of the Fcγ-receptors (FcγR) by IgG complexes present in IVIg ^{5,6}. It has been shown recently that the IgG complexes present in IVIg could also inhibit phagocytosis by binding to the negative FcγRIIB ⁷. The modulation of the complement system has been observed both *in vitro* and *in vivo* ². IgG present in IVIg or immune complexes (IC) formed in vivo following infusion of IVIg can interact with complement components such as C1q and C3/C4 and thus reduce the amount of these molecules available to induce cell destruction and tissue damage ². In both mechanisms, the infusing IVIg has to contain or to induce the formation of IgG complexes, which can interact with the FcγR and complement proteins. There has been previous work on the characterization of IgG complexes present in IVIg. These studies have showed that although IVIg contained mainly monomeric IgG (> 95%) it also contained a small but significant proportion of IgG complexes, which could be involved in the *in vivo* inhibitory effect on phagocytosis ⁸. These IgG complexes could be due to idiotype (id)-anti-id interactions in the IVIg caused by the blending of thousands of plasma donations from different individuals ^{9,10}. An alternative cause could be the industrial fractionation process, which could induce the formation of IgG aggregates ⁸. It is likely that the therapeutic IVIg component in autoimmune and inflammatory diseases represents only a small proportion of the injected material. This hypothesis is consistent with the very large doses of IVIg (e.g. 1-2 gr/kg), which are injected for the short-term therapy of several diseases (reviewed in¹¹). Characterization of the IVIg active components is important since it could permit to further fractionate the scarce IVIg preparations into different products for use in the treatment of diseases with different etiologies (e.g. immunodeficiencies and ITP).

It is now well recognized that the immune system of healthy individuals constantly produces IgM and IgG antibodies that can react with self-structures. These autoantibodies are part of the natural antibody (NA) class, which constitute a significant part of the serum antibodies ^{12,13}. NA are often polyreactive and can react with various self and non-self structures such as human and animal proteins present in serum, on cell surfaces or in cells, and other natural or synthetic chemical structures such as DNA, LPS, DNP, TNP, etc. They are thought to represent the first line of defense against infectious agents not previously encountered. Binding of the NA can result in phagocytosis of the infectious agent and lead to a protective immune response producing high affinity and monospecific IgG antibodies. For some unclear reasons, the mechanisms of control of the reactivity or production of autoimmune antibodies may get disregulated which can result in the development of various autoimmune and inflammatory diseases ¹². Under normal circumstances, the reactivity and production of serum IgG autoantibodies are tightly regulated in order to avoid formation of IC, which would result in inflammation. It was shown that the activities of autoreactive IgG were constantly inhibited by id-anti-id interactions with antibodies of the IgM class ¹⁴⁻¹⁶. This conclusion was derived from experiments in which the autoreactivity of IgG was shown to significantly increase after the removal of the inhibitory IgM present in serum by IgG purification. IVIg preparations contain mostly IgG (> 95%) with only trace amounts of IgM, IgA and other plasma proteins.

The prior art contains reports of studies investigating the mechanisms of action of IVIg through the preparation of diverse fractions and compositions thereof. For example, Vassiliev et al. (J. Autoimmunity, 1995, 8:405-423) have characterized IVIg fractions enriched in IgG dimers. Bourrel et al. (EP application 1 059 088 A1 published December 13, 2000) have prepared an IVIg composition by selecting antibodies that are specific for the DNP hapten. Vassilev et al. (Blood, 1999, 93: 3624-3631) have prepared an IVIg fraction enriched in antibodies specific for the RGD modif. Kaveri et al. have prepared an IVIg composition with antibodies specific for the B07.75-84 peptide corresponding to a conserved motif of the HLA class I molecule.

It would be highly desirable to be provided with a method to purify autoantibodies from therapeutic intravenous immunoglobulin preparations (IVIg).

### SUMMARY OF THE INVENTION

In accordance with the present invention there is provided a method to purify autoantibodies from therapeutic intravenous immunoglobulin preparations (IVIg) using affinity chromatography on a ligand bound to a solid support, wherein the ligand used for affinity chromatography is composed of a mixture of proteins present in human serum other than IgG.

The preferred autoantibodies are selected for reactivity with soluble proteins of human serum.

More preferably, the ligand used for affinity chromatography is composed of purified individual serum proteins, such as ferritin.

Another preferred ligand used for affinity chromatography is composed of animal proteins or other molecules which can be recognized by the autoantibodies.

A preferred solid support used for affinity chromatography is Sepharose or an equivalent thereof.

Another embodiment of the method of the present invention further comprises a step of recovering non-autoreactive antibodies for further processing in a flow-through fraction of the affinity chromatography column.

In accordance with another embodiment of the present invention there is provided autoantibodies isolated from therapeutic intravenous immunoglobulin preparations (IVIg) according to the method as defined above, wherein said antibodies are capable of forming autoimmune complexes in human serum wherein the autoimmune complexes are capable of binding to and activating complement in human serum.

In accordance with another embodiment of the present invention there is provided the use of autoantibodies of the present invention for the preparation of a medicament in the treatment of autoimmune and inflammatory disorders.

In accordance with another embodiment of the present invention there is provided a pharmaceutical composition for the treatment of autoimmune and inflammatory disorders in a patient, which comprises a therapeutically effective amount of autoantibodies of the present invention in association with a pharmaceutically acceptable carrier.

In accordance with another embodiment of the present invention there is provided autoantibodies-free therapeutic intravenous immunoglobulin (IVIg) preparation, which is substantially free of autoantibodies obtained by the method as defined above, where fractions enriched in auto-antibodies and fractions free of auto-antibodies are obtained from the affinity chromatography, characterized in that fractions substantially free of auto-antibodies are selected.

In accordance with another embodiment of the present invention there is provided a pharmaceutical composition for the treatment of immunodeficiency in a patient, which comprises a therapeutically effective amount of the autoantibodies-free therapeutic intravenous immunoglobulin (IVIg). Optionally, the pharmaceutical composition may further comprise a protein.

In accordance with another embodiment of the present invention there is provided the use of the autoantibodies-free IVIg for the preparation of a medicament in the treatment of immunodeficiency.

For the purpose of the present invention the following terms are defined below.

The term " autoimmune and inflammatory disorders " is intended to mean a group of multiple diseases characterized by an autoimmune reaction to the patient cells or tissues which may be accompanied by an inflammatory response due to the activation of the complement. IVIg are used in the treatment of several of these diseases ^{1,2,3}.

The term "immunodeficiency" is intended to mean the inability of an individual to produce enough immunoglobulins to remain healthy. The immunodeficiency can be primary (no obvious causes) or secondary to another disease (AIDS, cancer), which impairs the production of immunoglobulins by immune cells. Immunodeficiency is routinely treated by monthly injection of IVIg.

The term "polyreactive autoantibodies" is intended to mean antibodies produced by the immune system of an individual, which can recognize several structures present in the body of the individual ^{1,2}. Polyreactive autoantibodies are produced in all individuals and deregulation of their production or of their inhibition can lead to the development of autoimmune and inflammatory diseases.

### BRIEF DESCRIPTION OF THE DRAWINGS

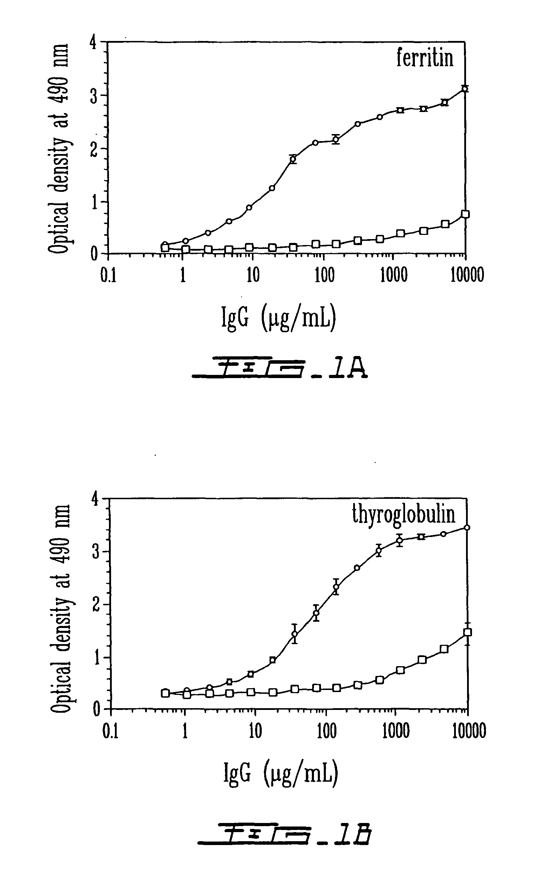

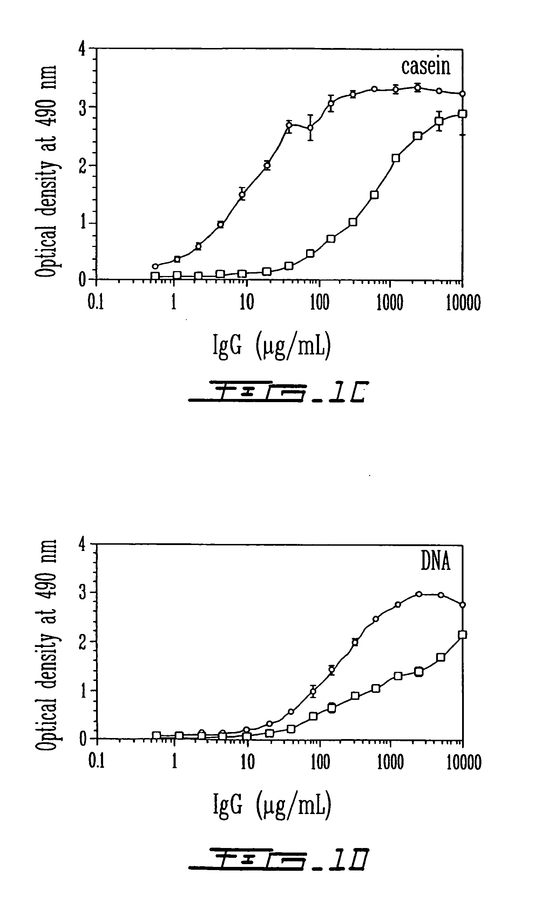

**Figure 1****.** Reactivity of IVIg and human serum for human ferritin (A), human thyroglobulin (B), bovine casein (C) and DNA (D). Reactivity of IgG content from IVIg (•) and human serum (■) was measured by polyreactive ELISA.

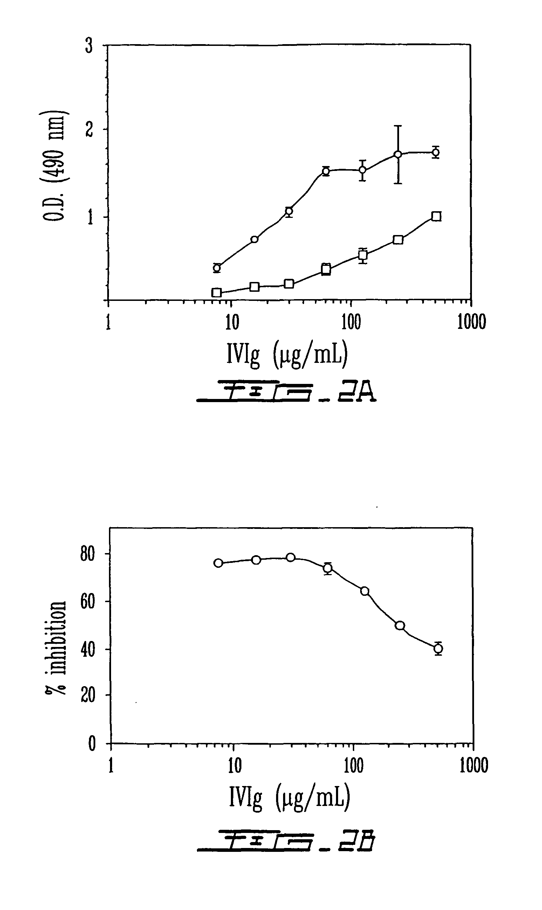

**Figure 2****.** Autoreactivity of IVIg for human ferritin in presence of serum. Panel A: ELISA reactivity of IVIg for ferritin in absence (●) and in presence of serum (25 µg/mL of IgG; ■). Panel B: Inhibition of ferritin reactivity of IVIg in presence of human serum.

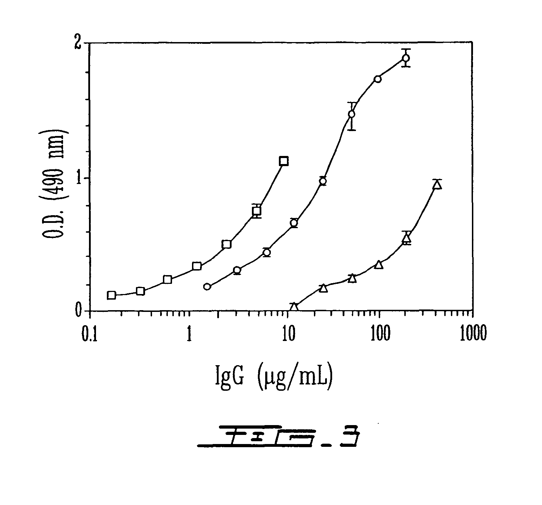

**Figure 3****.** ELISA reactivity of serum proteins-Sepharose fractions for ferritin. IVIg (●), eluate (■) and flow through (▲) fractions were serially diluted and tested in the ferritin-specific ELISA.

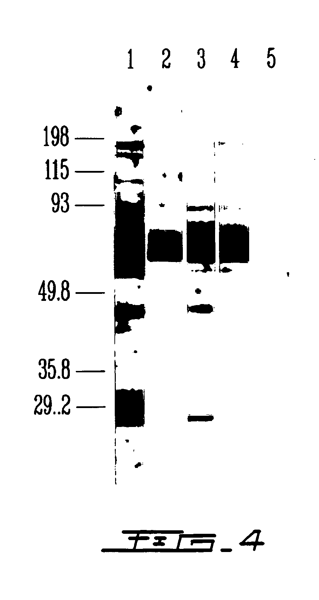

**Figure 4****.** Diversity of plasma proteins recognized by autoantibodies. IgG-depleted serum proteins were separated by SDS-Page and tested in Western blot experiments with total IVIg (lane 1, 6.25 µg/mL IgG), flow-through (lane 2, 6.25 µg/mL IgG), SP-Sepharose eluate (lane 3, 0.625 µg/mL IgG) and normal serum (lane 4, 6.25 µg/mL IgG). Negative control (without IgG) shows the absence of reactivity of the anti-human IgG-HRP conjugate.

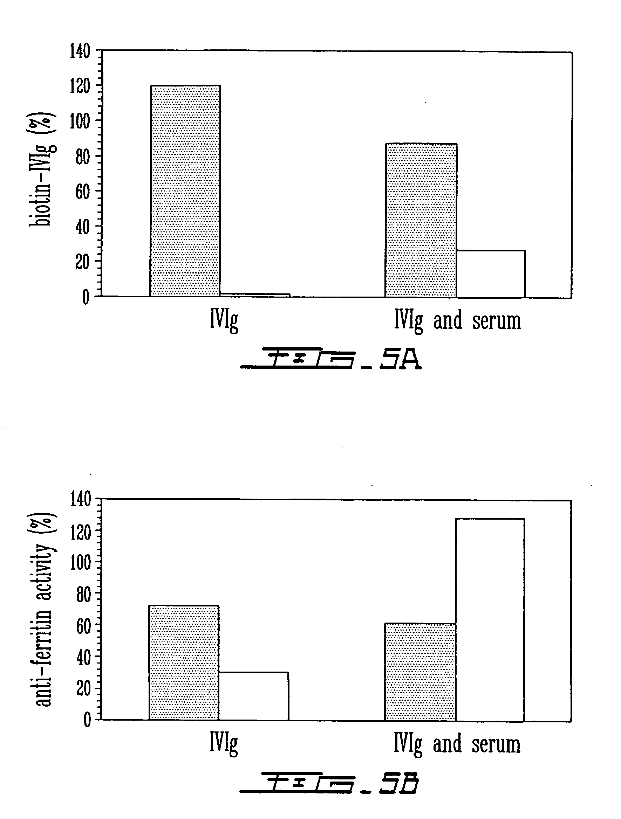

**Figure 5****.** Analysis of IC isolated by 2.5% PEG precipitation. The PEG supernatants (grey bars) and precipitates (clear bars) obtained after PEG treatment of biotin-IVIg alone or biotin-IVIg-serum blend were analyzed by ELISA for IgG content (upper panel) and anti-ferritin reactivity (lower panel). Only the biotin-IVIg were detected by using a streptavidin-HRP conjugate. The results are expressed as a percentage of the IgG and anti-ferritin activity present in the untreated preparations.

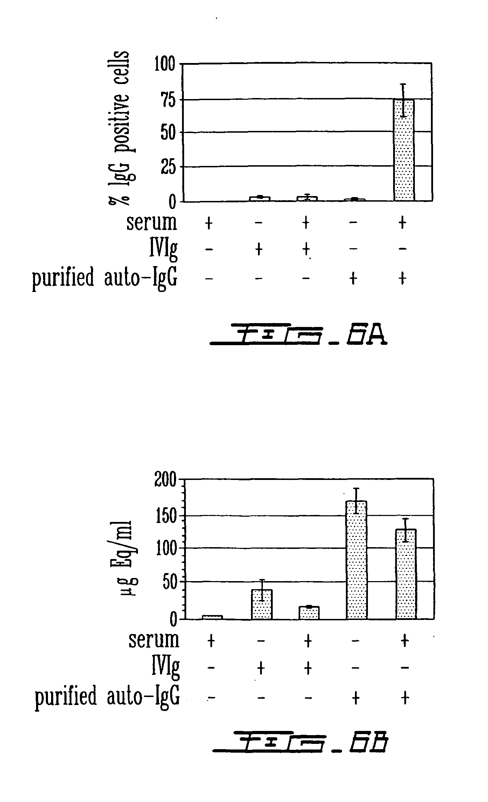

**Figure 6****.** Interaction of autoIC with human complement. IVIg and purified autoantibodies were added to human serum and the binding of complement was detected using the Raji cell complement receptor 2 (CR2) assay (Panel A) and the C1q binding assay (Panel B). The results are expressed as the percentage of IgG positive Raji cells (Panel A) and µg equivalent per mL (Panel B).

### DETAILED DESCRIPTION OF THE INVENTION

In the present work, we have tested whether the injection of large amounts of IVIg could overload the normal mechanisms of control of autoreactive IgG present in human plasma and result in transient formation of autoimmune complexes (autoIC). The results obtained support the hypothesis since we could detect the presence of autoIC in human serum containing therapeutic concentrations of IVIg.

Intravenous immunoglobulins (IVIg) are widely used in the treatment of several diseases. Its mechanisms of action in most autoimmune diseases are still not known but inhibition of phagocytosis and of complement activation have been documented. The origin of the responsible immune complexes (IC) is still unclear. We have studied the possibility that the addition of IVIg to serum could result in the formation of soluble IC due to the inability of the serum anti-idiotype IgM to inhibit the large amounts of infused autoantibodies present in IVIg. The results showed that serum could inhibit the anti-ferritin reactivity of IVIg up to a dose corresponding to two times the amount of endogenous serum IgG. The autoantibodies could be purified from IVIg by chromatography on serum-proteins Sepharose. IVIg and purified autoantibodies recognized a wide variety of serum proteins in Western blot experiments and were present in IC isolated from serum-IVIg blends. The autoantibodies and derived autoIC interacted with complement components as determined by the Raji cell and C1q binding assays. These results support a role of autoantibodies in the inhibition of phagocytosis and of complement activation induced by IVIg. The easy purification of the autoantibodies could permit to fractionate the current IVIg preparation into two products for use in the treatment of different diseases.

### Materials and methods

### Reagents

Human ferritin and thyroglobulin were purchased from Calbiochem (LaJolla, CA) and casein, from BDH Laboratories (Toronto, Ont.). Other antigens were provided from Sigma (Oakville, Ont.). Human serum was prepared from blood of healthy individuals after informed consent.

### Purification of polyspecific autoantibodies from IVIg

Serum was depleted of IgG by passage over a column of protein G-Sepharose (Life Technologies, Burlington, Ont., Canada). The IgG-depleted serum was dialyzed against 137 mM NaCl in 10 mM phosphate buffer, pH 7.4 (PBS) and the proteins were coupled to CNBr-activated Sepharose (Amersham-Pharmacia, Baie d'Urfé, Qc, Canada) as described by the supplier. IVIg (Gamimmune N 10%, Bayer Corporation, Toronto, Ont., Canada) were incubated overnight at room temperature with the serum proteins-Sepharose. After washing with PBS, bound IgG were eluted with 100 mM glycine-HCl, pH 2.5. The protein-rich fractions were dialyzed against 40 mM glycine pH 4.5 and concentrated using centrifugation in Centricon filter units (Millipore, Nepean, Ont., Canada). Quantification of total protein was done with the Bradford assay (BioRad, Mississauga, Ont., Canada) and the IgG content was determined by quantitative ELISA.

### Polyreactivity ELISA

Antigens were coated at 10 µg/mL, except for dsDNA and histone (50 µg/mL), in 100 mM carbonate buffer, pH 9.7 overnight at 4°C. Uncoated sites were blocked with 5 % bovine serum albumin (BSA) in 0.05 % Tween 20-PBS for 1 hour at 37°C. After washes with 0.85% saline, samples were diluted in 1 % BSA- 0.05 % Tween 20-PBS and distributed into wells for 1 hour at 37°C. Bound antibodies were then conjugated with peroxydase-labelled goat anti-human IgG (Fc specific; Jackson ImmunoResearch Laboratories, West Groove, PA) and revealed with o-phenyldiamine (OPD) reactive (Abbott Laboratories; Abbott Park, IL). Optical densities (OD) were read at 490 nm with a reference wavelength of 635 nm.

For competitive ELISA assays, we used the same technique and added a pre-incubation of samples to be tested.

### Polyreactive immunoblot

IgG-depleted serum (10 µg of protein per strip) was subjected to SDS-Page (10% polyacrylamide) and transferred on PDVF membrane (Millipore, Nepean, Ont.). Membrane was then incubated with 5% BSA in 100 mM NaCl, 10mM Tris-HCl, pH 7.4 (TBS) buffer for 60 minutes. After washes with TBS buffer, incubations with the different preparations diluted in 5% BSA-TBS buffer were performed during 60 minutes. PVDF membrane was incubated with HPR-conjugated mouse anti-human IgG (Fc specific; Southern Biotech, Birmingham, AL) for 60 minutes and finally revealed with ECL (Fisher, Nepean, Ont.).

### Isolation ofIC

IC were isolated according to a previously published method ^{17,18}. Briefly, samples were prepared and diluted in PBS. An equal volume of 5% PEG 6000 (Sigma) was added to the diluted sample and incubated overnight at 4°C. The precipitate was then isolated by centrifugation (1500x *g*; 20 minutes, 4°C), washed twice with 2.5% PEG 6000 and dissolved by incubation for 30 minutes at 37°C in PBS containing 0.05% Tween 20, 10 mM EDTA and 0.01% thimerosal. IgG content was then determined by quantitative ELISA and reactivity for various antigens, by polyreactive ELISA.

### Complement binding assays

The Raji cell assay which measures the complement-dependent interaction of IgG with the complement receptor 2 (CR2) was performed as previously described ²⁰. Briefly, Raji cells (1 X 10⁶ cells in 1 mL of PBS-glucose) were incubated for 45 minutes at 37°C with 12.5 µl of IVIg (6 mg/mL) or purified auto-IgG (150 µg/mL) in presence and absence of freshly thawed human serum (diluted to contain 6 mg/mL of IgG). Cells were then washed twice and labelled with a FITC-mouse anti-human IgG (Fc specific; BD Biosciences) for 15 minutes at 4°C. Cells were fixed in 2 % paraformaldehyde before the determination of the percentage of IgG positive cells by flow cytometry analysis (FASCalibur; Becton-Dickinson, Frankin Lakes, NJ). The binding of IC present in the above fractions to immobilized C1q was performed using the CIC-EIA kit and the AGH controls from Quidel (San Diego, CA) following the manufacturer's instructions. The relative results are expressed as µg equivalent per mL as described by the manufacturer.

### Results

### Comparative polyreactivity of IVIg and human serum

Purified IgG have been previously shown to be more polyreactive than corresponding amounts of human serum ¹⁵. To confirm this finding in our experimental system, we compared the polyreactivity of IVIg and human serum in ELISA done with similar IgG amount (25 µg/mL). The results (Table 1) indicated that the polyreactivity of the two preparations differed significantly. While the reactivity of IVIg and serum with α2-acid glycoprotein, thyroglobulin, casein, transferrin and LPS was similar, IVIg reacted much more strongly with other antigens such as ferritin, actin, dsDNA and KLH. The low reactivity of the human serum sample with these antigens was confirmed using sera prepared from the blood of three other donors. This result confirmed previous ones and showed that the components (i.e. IgM), which inhibit the reactivity of autoreactive IgG in serum, are not present in significant amounts in IVIg preparations.

**Table 1**

| **Comparative polyreactivity of human serum and IVIg** | | | | |
|---|---|---|---|---|
| | ELISA reactivity | | | |
| | | | Purified autoantibodies | |
| Target | serum | IVIg | Polyspecific | Ferritin-specific |
| ***Human proteins*** | | | | |
| α2-acid glycoprotein | ± | ± | ± | +++ |
| ferritin | 0 | +++ | +++ | +++ |
| fibronectin | 0 | 0 | ± | +++ |
| thyroglobulin | 0 | ± | ++ | ++ |

| ***Animal proteins*** | | | | |
|---|---|---|---|---|
| actin | ± | ++++ | ++++ | ++++ |
| casein | +++ | ++++ | ++++ | ++++ |
| histone | ± | ++ | +++ | ++++ |
| transferrin | 0 | ± | +++ | +++ |

| ***Others*** | | | | |
|---|---|---|---|---|
| dsDNA | ± | +++ | ++++ | ++++ |
| KLH | + | ++++ | ++++ | ++++ |
| LPS | +++ | +++ | +++ | +++ |

| | | | | |
|---|---|---|---|---|
| Polyreactivity was evaluated by ELISA using 25 µg/mL of IgG present in IVIg and human serum. The reactivity was quantified and compared to a scale of polyreactivity established as the following: ++++, more than 50 times the background O.D.; +++, between 10 and 50 times the background O.D.; ++, between 7 and 10 times the background O.D.; +, between 5 and 7 times the background O.D.; ±, between 2 and 5 times the background O.D. and; 0, below 2 times the background O.D. | | | | |

In additional experiments, we compared the relative reactivity of IVIg and human serum with soluble human plasma proteins (ferritin and thyroglobulin) and with other antigens (bovine casein and DNA). The results (Figure 1) are expressed as dose response curves for concentrations of IgG between 0.5 µg/mL and 10 mg/mL. The results indicated that the relative differences in reactivity between IVIg and human serum are much more important for soluble human plasma proteins (Figure 1 A, ferritin, and 1B, thyroglobulin) than for the two other antigens (Figure 1C, casein and 1D, DNA). Indeed, IVIg were about 1000 fold more reactive for ferritin than human serum. This ratio was about 250 for thyroglobulin but only 60 for casein and about 10 for DNA. This difference between the human antigens and the others indicated that the mechanisms controlling the polyreactivity of serum antibodies are much more effective against autoreactive antibodies than against polyreactive antibodies recognizing antigens not normally present in human plasma (e.g. casein and DNA). In the following experiments, we used purified human ferritin as an antigen model for all the other ones that are present in human plasma.

### Inhibition of IVIg anti-ferritin reactivity by human serum

We determined the ability of a fixed volume of human serum (25 µg/mL of IgG) to inhibit the reactivity of increasing amounts (10 to 500 µg/mL) of IVIg. The observation that the serum exhibited a very low anti-ferritin reactivity at 25 µg/mL of IgG (Figure 1) permitted to focus on the IVIg reactivity. Results are shown on Figure 2. In panel A, the OD results indicated that the addition of the fixed amount of serum to increasing doses of IVIg resulted in a significant reduction of the anti-ferritin reactivity at all IVIg doses tested. The shapes of the curves obtained suggested that the inhibition was more important at lower doses of IVIg. Indeed, the results when expressed as a percentage of inhibition by serum at each IVIg dose (Figure 2B) showed that the inhibition was high (> 75%) up to a dose of IVIg of about 50 µg/mL of IVIg was added. The inhibition then gradually decreased to reach a percentage of 40% at the maximal dose of IVIg tested (500 µg/mL). These results confirmed the ability of human serum to inhibit autoantibodies ¹⁶ and further indicated that the ability of the serum to control exogenously added IgG has limits. The saturating curve (Figure 2B) indicated that the amount of serum IgG can be increased by a factor of about 3 (25 µg/mL of endogenous IgG versus 50 µg/mL of exogenous IgG) before starting to detect a significant *in vitro* autoreactivity of the serum IVIg blend.

### Purification of autoantibodies reacting with human serum proteins

In preliminary experiments, we observed that the small proportion of the antibodies (about 1%) reacting with bovine casein could be easily purified from IVIg using affinity chromatography on columns of casein-Sepharose. For direct relevance to human patients, human serum was depleted of IgG by chromatography on protein G-Sepharose. The serum proteins were cross-linked in bulk to CNBr-activated Sepharose, which was used to purify the autoantibodies present in IVIg. Although the procedure should purify all the autoantibodies to plasma proteins present in IVIg, we used for the assay of the fractions (total, flow-though and column eluate) the ferritin ELISA. Representative results for the anti-ferritin activity of the various fractions are shown in Figure 3. The affinity chromatography resulted in a very significant depletion of ferritin autoantibodies as shown by the shift of the flow-through curve to the right. Conversely, the ferritin autoantibodies were enriched in the eluate fraction as shown by the shift to the left. The observation that the curves had nearly linear dose-response regions at low OD values (< 0.4) permitted the calculation of the purification results that are listed in Table 2.

**Table 2**

| **Purification of autoreactive IgG** | | | | | |
|---|---|---|---|---|---|
| Fractions | Total IgG (mg) | Total reactivity (U) | Specific activity (U/mg) | Purification (X) | Yield (%) |
| Starting IVIg | 14.24 | 172606 | 12121 | - | - |
| Flowthrough | 11.30 | 13831 | 1224 | 0.10 | 8.0 |
| Eluate | 0.42 | 136197 | 327869 | 27.05 | 78.9 |

| | | | | | |
|---|---|---|---|---|---|
| IVIg were chromatographied on a column of Sepharose coupled to human serum proteins. The flow through and glycine pH 2.5 eluate fractions were recovered and analyzed for their IgG content. Quantification of reactivity was estimated using ELISA and human ferritin as coating antigen. One unit of reactivity represents the amount of IgG needed to obtain an O.D. of 0.4. | | | | | |

As indicated by the ELISA results of Figure 3, the chromatography depleted the IVIg of more than 90% of the anti-ferritin activity. The glycine-HCl eluate contained about 3% of the starting IgG but more than 75% of the anti-ferritin activity. The calculated purification factor (27 X) is only indicative since it is likely much higher due to the fact that the IgG present in the eluate are expected to react with several plasma proteins and not only with ferritin. Thus the autoantibodies in IVIg that react with serum proteins can be greatly enriched by affinity chromatography.

### Diversity of serum proteins recognized by autoantibodies

To evaluate the diversity of soluble auto-antigens present in serum, we performed Western blot experiments with proteins present in IgG-depleted serum. The serum protein blots were probed with the various fractions and the binding of antibodies was detected with an anti-human IgG conjugate. The results are shown on Figure 4. We had to perform optimisation experiments to reduce the high background level, which was observed with the IVIg fraction. The results obtained with IVIg showed the presence of major bands of different molecular weights along with a diffuse staining of the blot suggesting the presence of many minor autoantigens. The intense band of 65 kilodaltons observed with all tested antibody samples corresponds to albumin and is most likely due to non-specific binding caused by the high albumin content of the blotted proteins. The affinity chromatography was effective in depleting the autoantibodies as seen by the absence of several bands and the clearer background. The purified autoantibodies reacted with several autoantigens with a pattern similar to the starting IVIg. Finally as expected from the ELISA results, serum IgG react only weakly with some of the separated proteins giving a pattern similar to the flow-through fraction of the affinity column. This analysis showed that the autoantibodies present in IVIg and in the affinity column eluate can interact with multiple proteins in human serum in contrast to the low reactivity of human serum.

### Soluble serum IC in presence of exogenous IVIg

The findings that the ferritin reactivity of IVIg was strongly inhibited by the presence of serum (Figure 2) and that IVIg reacted with multiple plasma proteins as detected in Western blot experiments (Figure 4) suggested the formation of IC in mixtures of serum and IVIg. This possibility was tested directly by precipitating the IC in presence of 2.5% PEG as previously reported ^{17,19}. In preliminary experiments, we used IgG-depleted serum to ensure that the IgG present in IC originated from the added IVIg. Although the addition of serum strongly inhibited the reactivity of IVIg, the remaining reactivity of IVIg was mostly (> 60%) found in the PEG precipitate indicating that the added IVIg could form IC with serum proteins. A similar result was obtained with purified autoantibodies. To rule out the possibility that these results were caused by the absence of endogenous serum IgG, the experiment was repeated with serum and biotinylated IVIg. The total and ferritin-reactive biotin-IVIg were assayed using a streptavidin conjugate in order to detect only the added IVIg. The results (Figure 5) first showed that the PEG treatment did not precipitate much IgG in the starting IVIg preparation (< 2 %). However, almost 20 % of the IVIg added to the serum was found in the PEG precipitate. As to the ferritin reactivity, most of the anti-ferritin IgG were found in the PEG supernatant of IVIg. In the experimental conditions used, the addition of serum resulted in a 50 % inhibition of IVIg reactivity with ferritin. But the PEG precipitate contained almost two times more ferritin reactivity. This result indicated the formation of soluble IC containing exogenously added IVIg in mixtures of serum and IVIg. A consistent observation in these experiments was the fact that the combined ferritin reactivity of the two PEG fractions was higher that the one of the starting serum-IVIg mixture. This result suggested that the reactivity of the soluble IC with ferritin was increased following their isolation by PEG treatment.

### Interactions of IVIg and purified auto-IgG with complement components in presence of human serum

The soluble IC formed in human serum by IVIg and purified auto-IgG could interact with complement components and consequently reduce the amount of complement components available for pathogenic effects. The interaction with complement components was studied using two established assays. The Raji cell CR2 assay measures the cell uptake of IC through the CR2. The results obtained (fig 6, panel A) indicated a very low percentage (<4%) of IgG-positive cells after incubation with either serum, IVIg, purified auto-IgG or a mixture of IVIg and serum. However incubation in presence of a mixture of purified auto-IgG and serum resulted in strongly IgG positive Raji cells (about 75 %) indicating that the ability of the auto-IgG-containing IC to interact with complement components is significantly higher that the one of the IC formed with IVIg. The commercial C1q binding assay measures the relative amount of IgG complexes that can bind to immobilized C1q. The results obtained with the above fractions (fig. 6, panel B) showed the binding of a significant amount of IgG in presence of IVIg alone and of the mixture of IVIg and serum. However the presence of the auto-IgG fraction resulted in much more bound IgG (4-6 X). The binding of an even higher amount of IgG in presence of isolated IVIg and auto-IgG fractions indicated that the polyreactive IgG present in IVIg and purified auto-IgG may directly bind the C1q molecule. Finally it should be pointed out that the higher reactivity of the purified auto-IgG in the two assays compared to IVIg was obtained in presence of a 40 times lower dose of IgG with the purified auto-IgG fraction (0,15 mg/mL versus 6 mg/mL for IVIg).

### Polyspecificity of ferritin autoantibodies

To determine if the autoantibodies reacting with various serum proteins (Figure 4) and present in IC (figure 5) are polyspecific or represent a mixture of more monospecific antibodies, we purified the ferritin-specific autoantibodies from IVIg using chromatography on ferritin-Sepharose. The procedure resulted in a 100-fold purification of ferritin autoantibodies and was efficient since the flow through fraction contained less than 5 % of the ferritin antibodies present in the starting IVIg. The polyspecificity of the purified anti-ferritin and anti-serum proteins was compared in ELISA using the antigen panel used above. The results (Table I) showed that the purified anti-serum proteins had a pattern of reactivity similar to the starting IVIg. The ferritin-specific autoantibodies reacted strongly with all tested structures including three other human serum proteins (α2-acid glycoprotein, fibronectin and thyroglobulin). This polyspecific reactivity was confirmed in Western blot experiments in which we observed, with anti-ferritin autoantibodies, a pattern of bands similar to the ones obtained with IVIg and purified anti-serum proteins (Figure 4). Thus, the ferritin autoantibodies are polyspecific indicating that they could form IC containing several serum proteins.

### Discussion

Our results show that the addition to human serum of doses of IVIg similar to the ones observed in the plasma of IVIg-treated patients, resulted in the formation of IC with soluble plasma proteins. These IC were apparently formed because the added amounts of purified IgG exceeded the ability of serum IgM to inhibit the autoreactive IgG through id-anti-id interactions. These reactive autoantibodies could be conveniently purified from IVIg through affinity chromatography on immobilized serum proteins or ferritin. Furthermore, the soluble IC were shown in *in vitro* assays to interact with complement proteins. Additional work is necessary to better characterize the biological activity of the autoantibodies but these results permit to draw some conclusions about the possible involvement of the autoIC in the modes of action of IVIg in diseases characterized by modulation of FcγR functions and of complement activation.

Previous work on the modulation of FcγR functions by IVIg has been focussed mainly on id-anti-id interactions, which could lead to the formation of IgG complexes ¹⁴⁻¹⁶. Our finding that such complexes could also be formed by interaction of autoantibodies and soluble plasma proteins reveals an additional source of IgG complexes, which could have increased FcγR modulating activity. Indeed the autoIC are expected to have a larger size and contain several IgG molecules for more efficient interaction with FcγR. Additional structural characterization of the autoIC will permit to confirm this hypothesis. Plasma IC have been observed in many autoimmune diseases ¹⁹ but the possible formation of soluble IC containing IVIg and plasma proteins has not been much studied so far. It is possible that these IC are rapidly cleared from circulation after interaction with FcγR-bearing cells. However, there is evidence that autoIC may be involved in the therapeutic effects of IVIg in some diseases. The reactive macrophage activation syndromes are characterized by a massive increase in plasma ferritin level (up to 10 mg/mL instead of < 1 µg/mL in healthy individuals). It was recently reported that the successful treatment of this disease by injection of large doses of IVIg (0,5-1 gr/kg) was related to the immune clearance of ferritin, which could be detected in plasma IC the day after IVIg injection ²¹. Our results on the inhibition of IVIg autoreactivity by serum support an involvement of autoIC in the mode of action of IVIg in other diseases. Large doses of IVIg (1-2 gr/kg) are used in the treatment of autoimmune and inflammatory diseases (reviewed in ¹¹). Our results are in agreement with a previous study ¹⁵ showing that the autoantibody inhibitory IgM present in serum must first be saturated before exogenously added IgG can form autoIC. The results (Figure 2) indicated that the autoreactivity of added IVIg is detected only after addition of a dose of IVIg containing about two times the endogenous amount of serum IgG. At this ratio, it is expected that the formation of autoIC would be optimal since further addition of autoantibodies results in proportional increase in ELISA reactivity of the serum-IVIg blend. The observed 2:1 proportion is similar to the plasma IgG increase observed in patients treated with about 1 gr/kg of IVIg.

Modulation of complement activation by IVIg has been shown to play a role in the therapeutic effect of IVIg in several inflammatory diseases. The results obtained in the *in vitro* assays indicate that the autoantibodies present in IVIg may play a role in this modulation. Indeed in the Raji cell binding assay which detects the presence of complement components in IgG complexes, only the mixture of purified autoantibodies and serum was highly reactive indicating that the soluble autoIC formed may interact with complement components. The results of the C1q assay showed the strong binding of purified autoantibodies in presence and absence of serum. It remains to be seen if the binding in absence of serum is due to the presence of IgG complexes in purified autoantibodies or to the recognition of the C1q molecule by the polyreactive autoantibodies. A consistent observation in the above assays was the increased reactivity of the purified autoantibodies compared with proportional amounts (40 times more IgG) of IVIg. The reason for this difference is unclear but it could indicate that the purification process removes some inhibitory molecules present in the IVIg preparations. The beneficial effects of IVIg in inflammatory diseases is thought to be dependent of its ability to scavenge complement fragments such as C3b and C4b, thus preventing their deposition in the tissue targeted by the pathogenic process ². The above results are consistent with this mechanism and further indicate that the autoantibodies present in IVIg may be involved in this process by interacting with activated complement components either directly or through the formed autoIC.

The chromatography of IVIg on immobilized serum proteins yielded an eluted fraction enriched in autoantibodies, which recognized a similar diversity of serum proteins on Western blots similar as the starting IVIg. The observation that purified ferritin autoantibodies are polyreactive and could bind to the three other serum proteins tested is significant in terms of the efficiency of the formation of IC after injection of IVIg. It suggests that the autoantibodies can rapidly form heterogeneous IC containing various plasma proteins. The high diversity of recognized plasma proteins also indicates that the formation of IC is less likely to result in immune depletion of certain plasma proteins in IVIg-treated patients. The purification results raise the interesting possibility of further fractionating the current IVIg preparations into two products. The flow-through of the column, which contains more than 95% of the starting IgG, is likely to represent IgG reacting with non-self structures and could be used to support immunodeficient patients. In this regard, the monthly infusion of IVIg in those patients is known to cause mild but significant adverse side effects in the first day following injection. It remains to be seen if removal of autoantibodies in IVIg could reduce the severity of these side effects. A rare but serious adverse effect of IVIg injection is anaemia resulting from the immune destruction of the patient red blood cells caused by the uptake of circulating IC by the complement receptor present on red blood cells ²². The origin of the pathogenic IC has remained unclear. It is tempting to speculate that these patients may have a reduced ability to inhibit a portion of the infused autoantibodies resulting in the formation of a higher amount of IC. The second fraction prepared by chromatography is the autoantibody eluate, which represents only about 3% of the starting IgG. This fraction could be useful in the treatment of the diseases in which IVIg have immunomodulatory roles or inhibit phagocytosis. Further characterization of the biological activity of the purified autoantibodies using *in vitro* (e.g. inhibition of phagocytosis ²³) and *in vivo* (e.g. passive murine model of ITP ²⁴) assays will permit to obtain the data, which could support the development of clinical trials in patients. These studies will reveal whether the non-autoreactive IgG present in the flow-through fraction are important for the formation of IC *in vivo.* It is possible that these IgG contribute in the saturation of the autoantibody inhibitory mechanisms present in serum. In this situation, the dose of autoantibodies necessary to obtain therapeutic effects could be proportionally much larger. However, preliminary works suggest that this is not the case since the amount of purified ferritin autoantibodies necessary to overcome the serum inhibition was found to be about 50 times less that the amount observed with starting IVIg (Figure 2). This observation indicated that, although the inhibitory anti-id present in serum are likely to be polyreactive, they are not able to inhibit all autoantibodies.

In conclusion, our results contribute to a better understanding of the mechanisms of action of IVIg in autoimmune and inflammatory diseases and could lead to refinements in the clinical use of IVIg through preparation of IVIg sub-products for different classes of diseases. This possibility would represent a significant advance in ensuring the future supply of IVIg, which is currently threatened by the continuous increase in clinical indications and market demand and by difficulties in collecting more donor-derived plasma for production of additional IVIg.

While the invention has been described in connection with specific embodiments thereof, it will be understood that it is capable of further modifications and this application is intended to cover any variations, uses, or adaptations of the invention following, in general, the principles of the invention and including such departures from the present disclosure as come within known or customary practice within the art to which the invention pertains and as may be applied to the essential features hereinbefore set forth, and as follows in the scope of the appended claims.

### REFERENCES

1. Kazatchkine MD, Kaveri SV. Immunomodulation of autoimmune and inflammatory diseases with intravenous immune globulin. N Eng J Med. 2001;345:747-755
2. Larroche C, Chanseaud Y, Garcia de la Pena-Lefebvre P, Mouthon L. Mechanisms of intravenous immunoglobulin action in the treatment of autoimmune disorders. Biodrugs. 2002;16:47-55
3. Ballow M. Intravenous immunoglobulins: clinical experience and viral safety. J Am Pharm Assoc. 2002;42:449-459
4. Lazarus AH, Freedman J, Semple JW. Intravenous immunoglobulin and anti-D in idiopathic thrombocytopenia purpura (ITP): mechanisms of action. Transfus Sci. 1998;19:289-294
5. Fehr J, Hofmann V, Kappeler U. Transient reversal of thrombocytopenia in idiotypathic thrombocytopenia purpura by high-dose intravenous gamma globulin. N Eng J Med. 1982;306:1254-1258
6. Kimberly RP, Salmon JE, Bussel JB, Crow MK, Hilgartner MW. Modulation of mononuclear phagocyte function by intravenous immunoglobulin. J Immunol. 1984;132:745-750
7. Samuelsson A, Towers TL, Ravetch JV. Anti-inflammatory activity of IVIg mediated through the inhibitory Fc receptor. Science. 2001;291:484-486
8. Matejtschuk P, Chidwick K, Prince A, More JE, Goldblatt E. A direct comparison of the antigen-specific antibody profiles of intravenous immunoglobulins derived from US and UK donors plasmas. Vox Sang. 2002;83:17-22
9. Dietrich G, Algiman M, Y S, Nydegger UE, Kazatchkine MD. Origin of anti-idiotypic activity against anti-factor VIII autoantibodies in pools of normal human immunoglobulin G (IVIg). Blood. 1992;79:2946-2951
10. Vassilev TL, Bineva IL, Dietrich G, Kaveri SV, Kazatchkine MD. Variable region-connected, dimeric fraction of intravenous immunoglobulin enriched in natural autoantibodies. J Autoimmun. 1995;8:405-413
11. Dahl MV, Bridges AG. Intravenous immune globulin: fighting antibodies with antibodies. J Am Acad Dermatol. 2001 ;45:775-783
12. Avrameas S, Ternynck T. The natural autoantibodies system: between hypotheses and facts. Mol Immunol. 1993;30:1133-1142
13. Lacroix-Desmazes S, Kaveri SV, Mouthon L, Ayouba A, Evelyne M, Coutinho A, Kazatchkine MD. Self-reactive antibodies (natural autoantibodies) in healthy individuals. J Immunol Methods. 1998;216:117-137
14. Adib M, Ragimbeau J, Avrameas S, Ternynck T. IgG autoantibody activity in normal mouse serum is controlled by IgM. J Immunol. 1990;145:3807-3813
15. Berneman A, Guilbert B, Eschrich S, Avrameas S. IgG auto- and polyreactivities of normal human sera. Mol Immunol. 1993;30:1499-1510
16. Rossi F, Guilbert B, Tonnelle C, Ternynck T, Fumoux F, Avrameas S, Kazatchkine MD. Idiotypic interactions between normal human polyspecific IgG and natural IgM antibodies. Eur J Immunol. 1990;20:2089-2094
17. Ohlson S, Zetterstrand K. Detection of circulating immune complexes by PEG precipitation combined with ELISA. J Immunol Methods. 1985;77:87-93
18. Lock RJ, Unsworth DJ. Measurement of immune complexes is not useful in routine clinical practice. Ann Clin Biochem. 2000;37:253-261
19. Louzir H, Ternynck T, Gorgi Y, Ayed K, Avrameas S. Enzyme immunoassay analysis of antibody specificities present in the circulating immune complexes of selected pathological sera. J Immunol Methods. 1988;114:145-153
20. Theofilopoulos AN. The Raji, conglutinin, and anti-C3 assays for the detection of complement-fixing immune complexes. Methods Enzymol. 1981;74:511-530
21. Emmenegger U, Frey U, Reimers A, Fux C, Semela D, Cottagnoud P, Spaeth PJ, Neftel KA. Hyperferritinemia as indicator for intravenous immunoglobulin treatment in reactive macrophage activation syndromes. AmJ Hematol. 2001;68:4-10
22. Kessary-Shoham H, Levy Y, Shoenfled Y, Lorber M, Gershon H. In vivo administration of intravenous immunoglobulin (IVig) can lead to enhanced erythrocyte sequestration. J Autoimmun. 1999; 13:129-135
23. Hadley AG, Kumpel BM, Merry AH. The chemiluminescent response of human monocytes to red cells sensitized with monoclonal anti-Rh(D) antibodies. Clin Lab Haematol. 1988;10:377-384
24. Mizutani H, Engelman RW, Kurata Y, Ikehara S, Good RA. Development and characterization of monoclonal antiplatelet autoantibodies from autoimmune thrombocytopenia purpura-prone (NZW x BXSB) F1 mice. Blood. 1993;82:837-844

## Claims

1. A method to purify autoantibodies from therapeutic intravenous immunoglobulin preparations (IVIg) using affinity chromatography on a ligand bound to a solid support, wherein the ligand used for affinity chromatography is composed of a mixture of proteins present in human serum other than IgG.

2. The method of claim 1, wherein the autoantibodies are selected for reactivity with soluble proteins of human serum.

3. The method of claim 1, wherein the ligand used for affinity chromatography is composed of purified individual serum proteins.

4. The method of claim 1, wherein the ligand used for affinity chromatography is composed of animal proteins or other molecules which can be recognized by the autoantibodies.

5. The method of claim 3, wherein the purified individual serum proteins comprise ferritin.

6. The method of claim 1, wherein the solid support used for affinity chromatography is Sepharose or an equivalent thereof.

7. The method of claim 1, which further comprises a step of recovering non-autoreactive antibodies for further processing in a flow-through fraction of the affinity chromatography column.

8. Autoantibodies isolated from therapeutic intravenous immunoglobulin preparations (IVIg) according to the method as defined in anyone of claims 1 to 7, wherein said antibodies are capable of forming autoimmune complexes in human serum.

9. The autoantibodies of claim 8, wherein the autoimmune complexes are capable of binding to and activating complement in human serum.

10. The use of autoantibodies of claim 9 for the preparation of a medicament in the treatment of autoimmune arid inflammatory disorders.

11. A pharmaceutical composition for the treatment of autoimmune and inflammatory disorders in a patient, which comprises a therapeutically effective amount of auto-antibodies of claim 9 in association with a pharmaceutically acceptable carrier.

12. An autoantibodie-free therapeutic intravenous immunoglobulin (IVIg) preparation, which is substantially free of autoantibodies obtained by the method of anyone of claims 1 to 7, where fractions enriched in auto-antibodies and fractions free of auto-antibodies are obtained from the affinity chromatography, **characterized in that** fractions substantially free of auto-antibodies are selected.

13. A pharmaceutical composition for the treatment of immunodeficiency in a patient, which comprises a therapeutically effective amount of an autoantibodies-free therapeutic intravenous immunoglobulin (IVIg) of claim 12.

14. The pharmaceutical composition of claim 13, which further comprises a protein.

15. The use of autoantibodies-free IVIg of claim 12 for the preparation of a medicament in the treatment of immunodeficiency.

## Patentansprüche

1. Verfahren zur Reinigung von Autoantikörpern aus therapeutischen intravenösen Immunoglobulinpräparaten (IVIg) unter Verwendung der Affinitätschromatographie an einem Liganden, der an ein festes Trägermaterial gebunden ist, wobei der Ligand, der zur Affinitätschromatographie verwendet wird, aus einer Mischung von Proteinen außer IgG zusammengesetzt ist, die in Humanserum vorhanden sind.

2. Verfahren nach Anspruch 1, wobei die Autoantikörper zur Reaktionsfähigkeit mit löslichen Proteinen von Humanserum ausgewählt sind.

3. Verfahren nach Anspruch 1, wobei der Ligand, der zur Affinitätschromatographie verwendet wird, aus einzelnen gereinigten Serumproteinen zusammengesetzt ist.

4. Verfahren nach Anspruch 1, wobei der Ligand, der zur Affinitätschromatographie verwendet wird, aus tierischen Proteinen oder anderen Molekülen zusammengesetzt ist, die von den Autoantikörpern erkannt werden können.

5. Verfahren nach Anspruch 3, wobei die einzelnen gereinigten Serumproteine Ferritin umfassen.

6. Verfahren nach Anspruch 1, wobei das feste Trägermaterial, das zur Affinitätschromatographie verwendet wird, Sepharose oder ein Äquivalent davon ist.

7. Verfahren nach Anspruch 1, welches ferner einen Schritt des Rückgewinnens von nichtselbstreaktionsfähigen Antikörpern zur Weiterverarbeitung in einer Durchflussfraktion der Affinitätschromatografiesäule umfasst.

8. Autoantikörper, die von therapeutischen intravenösen Immunoglobulinpräparaten (IVIg) gemäß dem Verfahren nach einem der Ansprüche 1 bis 7 isoliert sind, wobei die Antikörper zur Bildung von Autoimmunkomplexen in Humanserum fähig sind.

9. Autoantikörper nach Anspruch 8, wobei die Autoimmunkomplexe zur Bindung an und Aktivierung von Komplement in Humanserum fähig sind.

10. Verwendung von Autoantikörpern nach Anspruch 9 zur Zubereitung eines Medikaments in der Behandlung von Autoimmun- und Entzündungskrankheiten.

11. Pharmazeutische Zusammensetzung zur Behandlung von Autoimmun- und Entzündungskrankheiten bei einem Patienten, welche eine therapeutisch wirksame Menge von Autoantikörpern nach Anspruch 9 in Verbindung mit einem pharmazeutisch annehmbaren Träger umfasst.

12. Autoantikörperfreies therapeutisches intravenöses Immunoglobulin (IVIg)-Präparat, welches im Wesentlichen frei von Autoantikörpern ist und durch das Verfahren nach einem der Ansprüche 1 bis 7 gewonnen wird, wobei Fraktionen, die mit Autoantikörpern angereichert sind, und Fraktionen, die frei von Autoantikörpern sind, aus der Affinitätschromatographie gewonnen werden, **dadurch gekennzeichnet, dass** Fraktionen, die im Wesentlichen frei von Autoantikörpern sind, ausgewählt sind.

13. Pharmazeutische Zusammensetzung zur Behandlung von Immundefizienz bei einem Patienten, welche eine therapeutisch wirksame Menge eines autoantikörperfreien therapeutischen intravenösen Immunoglobulins (IVIg) nach Anspruch 12 umfasst.

14. Pharmazeutische Zusammensetzung nach Anspruch 13, welche ferner ein Protein umfasst.

15. Verwendung von autoantikörperfreiem IVIg nach Anspruch 12 zur Zubereitung eines Medikaments in der Behandlung von Immundefizienz.

## Revendications

1. Procédé de purification d'auto-anticorps à partir de préparations thérapeutiques d'immunoglobulines intraveineuses (IgIV) à l'aide d'une chromatographie d'affinité sur un ligand lié à un support solide, où le ligand utilisé pour la chromatographie d'affinité est composé d'un mélange de protéines présentes dans le sérum humain autres que des IgG.

2. Procédé selon la revendication 1, dans lequel les auto-anticorps sont choisis pour leur réactivité avec des protéines solubles du sérum humain.

3. Procédé selon la revendication 1, dans lequel le ligand utilisé pour la chromatographie d'affinité est composé de protéines sériques individuelles purifiées.

4. Procédé selon la revendication 1, dans lequel le ligand utilisé pour la chromatographie d'affinité est composé de protéines animales ou d'autres molécules qui peuvent être reconnues par les auto-anticorps.

5. Procédé selon la revendication 3, dans lequel les protéines sériques individuelles purifiées comprennent la ferritine.

6. Procédé selon la revendication 1, dans lequel le support solide utilisé pour la chromatographie d'affinité est le sépharose ou un équivalent de celui-ci.

7. Procédé selon la revendication 1, qui comprend en outre une étape consistant à récupérer les anticorps non autoréactifs pour un autre traitement dans une fraction non retenue de la colonne de chromatographie d'affinité.

8. Auto-anticorps isolés à partir de préparations thérapeutiques d'immunoglobulines intraveineuses (IgIV) selon le procédé tel que défini dans l'une quelconque des revendications 1 à 7, dans lequel lesdits anticorps sont capables de former des complexes auto-immuns dans le sérum humain.

9. Auto-anticorps selon la revendication 8, où les complexes auto-immuns sont capables de se lier à et d'activer le complément dans le sérum humain.

10. Utilisation des auto-anticorps selon la revendication 9 pour la préparation d'un médicament destiné au traitement de troubles auto-immuns et inflammatoires.

11. Composition pharmaceutique destinée au traitement de troubles auto-immuns et inflammatoires chez un patient, qui comprend une quantité thérapeutiquement efficace d'auto-anticorps selon la revendication 9 en association avec un support pharmaceutiquement acceptable.

12. Préparation thérapeutique d'immunoglobulines intraveineuses (IgIV) dépourvus d'auto-anticorps, qui est pratiquement dépourvue d'auto-anticorps obtenus ar le procédé selon l'une quelconque des revendications 1 à 7, où les fractions enrichies en auto-anticorps et les fractions dépourvues d'auto-anticorps sont obtenues à partir de la chromatographie d'affinité, **caractérisée en ce que** les fractions pratiquement dépourvues d'auto-anticorps sont choisies.

13. Composition pharmaceutique destinée au traitement d'une immunodéficience chez un patient, qui comprend une quantité thérapeutiquement efficace d'une préparation thérapeutique d'immunoglobulines intraveineuses (IgIV) dépourvues d'auto-anticorps selon la revendication 12.

14. Composition pharmaceutique selon la revendication 13, qui comprend en outre une protéine.

15. Utilisation d'IgIV dépourvues d'auto-anticorps selon la revendication 12 pour la préparation d'un médicament destiné au traitement d'une immunodéficience.
